(19) **Europäisches Patentamt / European Patent Office / Office européen des brevets**

(11) **EP 2 225 273 B1**

(12) **EUROPEAN PATENT SPECIFICATION**

(45) Date of publication and mention
of the grant of the patent:
**23.05.2012 Bulletin 2012/21**

(51) Int Cl.:
*C07K 16/06* [(2006.01)]     *G01N 33/68* [(2006.01)]

(21) Application number: **08864164.2**

(22) Date of filing: **18.12.2008**

(86) International application number:
**PCT/EP2008/010800**

(87) International publication number:
**WO 2009/080278 (02.07.2009 Gazette 2009/27)**

(54) **STABILITY TESTING OF ANTIBODIES**

STABILITÄTSPRÜFUNG VON ANTIKÖRPERN

ESSAI DE STABILITÉ D'ANTICORPS

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **21.12.2007 EP 07024863**

(43) Date of publication of application:
**08.09.2010 Bulletin 2010/36**

(73) Proprietor: **Roche Glycart AG
8952 Schlieren-Zuerich (CH)**

(72) Inventors:
• **ESER, Bianca
83670 Bad Heilbrunn (DE)**
• **KOLL, Hans
85247 Oberroth (DE)**
• **REGULA, Joerg, Thomas
80639 Muenchen (DE)**
• **SONDERMANN, Peter
82131 Stockdorf (DE)**

(74) Representative: **Burger, Alexander
Roche Diagnostics GmbH
Patent Department (TR-E)
Postfach 11 52
82372 Penzberg (DE)**

(56) References cited:
**WO-A-2007/024743**

• **RAJU ET AL: "Glycosylation in the Fc domain of IgG increases resistance to proteolytic cleavage by papain" BIOCHEMICAL AND BIOPHYSICAL RESEARCH COMMUNICATIONS, ACADEMIC PRESS INC. ORLANDO, FL, US, vol. 341, no. 3, 17 March 2006 (2006-03-17), pages 797-803, XP005274707 ISSN: 0006-291X**
• **VINCENTS BJARNE ET AL: "Enzymatic characterization of the streptococcal endopeptidase, IdeS, reveals that it is a cysteine protease with strict specificity for IgG cleavage due to exosite binding" BIOCHEMISTRY, vol. 43, no. 49, 14 December 2004 (2004-12-14), pages 15540-15549, XP002475900 ISSN: 0006-2960 cited in the application**
• **HESS ET AL: "Immunoglobulin cleavage by the streptococcal cysteine protease IdeS can be detected using protein G capture and mass spectrometry" JOURNAL OF MICROBIOLOGICAL METHODS, ELSEVIER, AMSTERDAM,, NL, vol. 70, no. 2, 10 July 2007 (2007-07-10), pages 284-291, XP022146176 ISSN: 0167-7012 cited in the application**
• **BECK A ET AL: "Characterization by liquid chromatography combined with mass spectrometry of monoclonal anti-IGF-1 receptor antibodies produced in CHO and NS0 cells" JOURNAL OF CHROMATOGRAPHY B: BIOMEDICAL SCIENCES & APPLICATIONS, ELSEVIER, AMSTERDAM, NL, vol. 819, no. 2, 25 May 2005 (2005-05-25), pages 203-218, XP004838690 ISSN: 1570-0232**

**(Cont. next page)**

**EP 2 225 273 B1**

- **LOO TREVOR ET AL: "Using secretion to solve a solubility problem: High-yield expression in Escherichia coli and purification of the bacterial glycoamidase PNGase F" PROTEIN EXPRESSION AND PURIFICATION, vol. 24, no. 1, February 2002 (2002-02), pages 90-98, XP002475901 ISSN: 1046-5928**

Sequence Listing

<110> F. Hoffmann-La Roche AG

<120> Stability testing of antibodies

<130> 24549 WO

<150> EP 07024863.8
<151> 2007-12-21

<160> 2

<170> PatentIn version 3.2

<210> 1
<211> 339
<212> PRT
<213> Streptococcus pyogenes

<400> 1

```
Met Arg Lys Arg Cys Tyr Ser Thr Ser Ala Ala Val Leu Ala Ala Val
1             5               10             15

Thr Leu Phe Val Leu Ser Val Asp Arg Gly Val Ile Ala Asp Ser Phe
            20              25             30

Ser Ala Asn Gln Glu Ile Arg Tyr Ser Glu Val Thr Pro Tyr His Val
        35              40              45

Thr Ser Val Trp Thr Lys Gly Val Thr Pro Pro Ala Asn Phe Thr Gln
    50              55              60

Gly Glu Asp Val Phe His Ala Pro Tyr Val Ala Asn Gln Gly Trp Tyr
65              70              75              80

Asp Ile Thr Lys Thr Phe Asn Gly Lys Asp Asp Leu Leu Cys Gly Ala
            85              90              95

Ala Thr Ala Gly Asn Met Leu His Trp Trp Phe Asp Gln Asn Lys Asp
        100             105             110

Gln Ile Lys Arg Tyr Leu Glu Glu His Pro Glu Lys Gln Lys Ile Asn
        115             120             125

Phe Asn Gly Glu Gln Met Phe Asp Val Lys Glu Ala Ile Asp Thr Lys
    130             135             140

Asn His Gln Leu Asp Ser Lys Leu Phe Glu Tyr Phe Lys Glu Lys Ala
145             150             155             160

Phe Pro Tyr Leu Ser Thr Lys His Leu Gly Val Phe Pro Asp His Val
            165             170             175
```

```
        Ile Asp Met Phe Ile Asn Gly Tyr Arg Leu Ser Leu Thr Asn His Gly
                    180             185             190


        Pro Thr Pro Val Lys Glu Gly Ser Lys Asp Pro Arg Gly Gly Ile Phe
                    195             200             205


        Asp Ala Val Phe Thr Arg Gly Asp Gln Ser Lys Leu Leu Thr Ser Arg
            210             215             220


        His Asp Phe Lys Glu Lys Asn Leu Lys Glu Ile Ser Asp Leu Ile Lys
        225             230             235             240


        Lys Glu Leu Thr Glu Gly Lys Ala Leu Gly Leu Ser His Thr Tyr Ala
                    245             250             255


        Asn Val Arg Ile Asn His Val Ile Asn Leu Trp Gly Ala Asp Phe Asp
                    260             265             270


        Ser Asn Gly Asn Leu Lys Ala Ile Tyr Val Thr Asp Ser Asp Ser Asn
                    275             280             285


        Ala Ser Ile Gly Met Lys Lys Tyr Phe Val Gly Val Asn Ser Ala Gly
            290             295             300


        Lys Val Ala Ile Ser Ala Lys Glu Ile Lys Glu Asp Asn Ile Gly Ala
        305             310             315             320


        Gln Val Leu Gly Leu Phe Thr Leu Ser Thr Gly Gln Asp Ser Trp Asn
                    325             330             335


        Gln Thr Asn
```

<210> 2
<211> 314
<212> PRT
<213> Flavobacterium ameridies

<400> 2

```
        Ala Pro Ala Asp Asn Thr Val Asn Ile Lys Thr Phe Asp Lys Val Lys
        1               5               10              15


        Asn Ala Phe Gly Asp Gly Leu Ser Gln Ser Ala Glu Gly Thr Phe Thr
                    20              25              30


        Phe Pro Ala Asp Val Thr Ala Val Lys Thr Ile Lys Met Phe Ile Lys
                    35              40              45
```

```
Asn Glu Cys Pro Asn Lys Thr Cys Asp Glu Trp Asp Arg Tyr Ala Asn
    50                  55                  60

Val Tyr Val Lys Asn Lys Thr Thr Gly Glu Trp Tyr Glu Ile Gly Arg
65              70                  75                      80

Phe Ile Thr Pro Tyr Trp Val Gly Thr Glu Lys Leu Pro Arg Gly Leu
            85                  90                      95

Glu Ile Asp Val Thr Asp Phe Lys Ser Leu Leu Ser Gly Asn Thr Glu
        100                 105                 110

Leu Lys Ile Tyr Thr Glu Thr Trp Leu Ala Lys Gly Arg Glu Tyr Ser
        115                 120                 125

Val Asp Phe Asp Ile Val Tyr Gly Thr Pro Asp Tyr Lys Tyr Ser Ala
    130                 135                 140

Val Val Pro Val Val Gln Tyr Asn Lys Ser Ser Ile Asp Gly Val Pro
145                 150                 155                 160

Tyr Gly Lys Ala His Thr Leu Ala Leu Lys Lys Asn Ile Gln Leu Pro
            165                 170                 175

Thr Asn Thr Glu Lys Ala Tyr Leu Arg Thr Thr Ile Ser Gly Trp Gly
        180                 185                 190

His Ala Lys Pro Tyr Asp Ala Gly Ser Arg Gly Cys Ala Glu Trp Cys
        195                 200                 205

Phe Arg Thr His Thr Ile Ala Ile Asn Asn Ser Asn Thr Phe Gln His
    210                 215                 220

Gln Leu Gly Ala Leu Gly Cys Ser Ala Asn Pro Ile Asn Asn Gln Ser
225                 230                 235                 240

Pro Gly Asn Trp Thr Pro Asp Arg Ala Gly Trp Cys Pro Gly Met Ala
            245                 250                 255

Val Pro Thr Arg Ile Asp Val Leu Asn Asn Ser Leu Ile Gly Ser Thr
        260                 265                 270

Phe Ser Tyr Glu Tyr Lys Phe Gln Asn Trp Thr Asn Asn Gly Thr Asn
        275                 280                 285

Gly Asp Ala Phe Tyr Ala Ile Ser Ser Phe Val Ile Ala Lys Ser Asn
    290                 295                 300

        Thr Pro Ile Ser Ala Pro Val Val Thr Asn
            305             310
```

22

**Description**

**Background information**

The pharmaceutical industry field has been very successful in recent years with products based among others on enzymes, antibodies and cytokines such as e.g. erythropoietin, interferons, plasminogen activators etc. The worldwide demand for protein therapeutic agents increases every year. Therapeutic monoclonal antibodies (mAbs, monoclonal antibodies) are an important group within the protein therapeutics. They are referred to as monoclonal because, in contrast to polyclonal antibodies, they are secreted by immune cells (cell clones) which are derived from a single antibody-forming cell. A characteristic of monoclonal antibodies is that they are each only directed against one epitope of an immunogenic substance and thus only against one antigenic determinant and can therefore be used very specifically in the treatment of diseases. Examples of protein therapeutics are the monoclonal antibodies Trastuzumab (commercial name: Herceptin), Daclizumab (commercial name: Zenapax) and Rituximab (commercial name: MabThera) from the Roche Diagnostics GmbH Company which have been used successfully for the treatment of among others breast cancer (Trastuzumab), for organ rejection (Daclizumab) and to treat non-Hodgkin lymphoma (Rituximab).

Therapeutic monoclonal antibodies are obtained by means of complex biotechnological processes. Degradation products may be formed during their production, formulation and storage which are often due to processes like oxidation and deamidation reactions as well as proteolytic cleavages (Yan, B., et al., J. Chromatog. A 1164 (2007) 153-161). Modifications of biological products may result in a change in the activity and/or immunogenicity due to structural changes in the molecule even if they only occur to a slight extent.

The quality of a biopharmaceutical product is of decisive importance in addition to its action. Therefore in addition to a detailed investigation of the modes of action, it is absolutely essential to determine the identity, purity and activity of protein-based drugs in order to use them safely as therapeutic agents.

Although mAbs can be successfully analysed by means of various separation and testing techniques, it has for a long time been difficult to apply and optimize RP-HPLC methods (RP-HPLC, Reversed Phase-High Performance Liquid Chromatography) to separate antibody species. However, various modifications of the antibody are often present simultaneously during the course of a degradation process, which makes it more difficult to analyse the diverse chromatographic and electrophoretic bands. Analysis by means of liquid chromatographic separation methods coupled with high resolution mass spectrometers (LC/MS, liquid chromatography/mass spectrometry) yields information about the exact mass of the various species and thus facilitates the identification of the antibody variants (Dillon, T.M., et al., J. Chromatogr. A, 1053 (2004) 299-305).

The cysteine endoprotease IdeS (Immunoglobulin degrading enzyme S) from the human pathogen Streptococcus pyogenes which is also referred to as Mac-1 or sib-38, is a cysteine protease that specifically cleaves the heavy chain of antibodies of the immunoglobulin G type (IgG). IgG is hitherto the only known substrate of IdeS (Vincents, B., et al., Biochem. 43 (2004) 15540-15549). IdeS consists of 339 amino acids including a signal peptide comprising 29 amino acids (von Pawel-Rammingen, U., et al., EMBO J. 21 (2002) 1607-1615) where an RGD motif is formed by the amino acids 214 to 216. IdeS cleaves human IgG (class G immunoglobulin) between the amino acids 236 and 237 (Gly-Gly) which are contained in the recognition sequence LLGGP. Human IgG2 is cleaved between the amino acids alanine and glycine in the recognition motif PVAGP. Murine antibodies of the IgG2a and IgG3 type are also cleaved (Vincents, B., et al., Biochem. 43 (2004) 15540-15549).

Hess, J.K., et al. (Hess, J.K., et al., J. Microbiol. Meth. 70 (2007) 284-291) report a mass spectroscopic method for determining the enzymatic activity of IdeS with the aid of SELDI-TOF mass spectrometry. A polypeptide which was isolated from S. pyogenes and has an IgG cysteine protease activity is reported in the US 2007/0237784. A method for forming Fc or Fab fragments of antibodies is reported in the European Patent Application EP 1 458 861. IdeS protease from group A streptococci is reported in WO 2006/131347.

Shantha Raju, T. and Scallon, B.J. report in Biochem. Biophys. Res. Commun. 341 (2006) 797-803 that glycosylation in the Fc domain of IgG increases resistance to proteolytic cleavage by papain. Characterization by liquid chromatography combined with mass spectrometry of monoclonal anti-IGF-1 receptor antibodies produced in CHO and NS0 cells is reported by Beck, A. et al. (J. Chrom. B 819 (2005) 2303-218). Loo, T., et al. (Prot. Expr. Purif. 24 (2002) 90-98) report using secretion to solve a solubility problem: high-yield expression in Escherichia coli and purification of the bacterial glycoamidase PNGase F.

**Summary of the invention**

The present invention describes a method for detecting IgG antibodies and IgG antibody fragments or modified forms of an IgG antibody in a sample, characterized in that it comprises the following steps:

a) providing a sample which contains an IgG antibody and/or its cleavage products,

b) incubating the sample provided under a) with

i) the IgG-specific cysteine protease IdeS,

ii) N-glycosidase F,

iii) the reducing agent trichloroethyl phosphate and formic acid,

whereby the incubation in steps b)-i), b)-ii) and b)-iii) is sequentially,

c) analysing the sample incubated under b) by means of a coupled liquid chromatography and mass spectrometry to detect the intact antibody and to detect fragments or modified forms of the antibody contained in the solution provided under a).

In one embodiment of the method the cysteine protease IdeS is derived from Streptococcus pyogenes or Treponema denticola. In yet a further embodiment the IgG-specific cysteine protease has the amino acid sequence SEQ ID NO:1. Another embodiment comprises incubation with an IgG-specific cysteine protease in the pH range between 5.5 and 8.5. In another embodiment the pH range is between pH 7.0 and 8.0 and in a further embodiment between pH 7.5 and 8.0. In yet a further embodiment the molar ratio of the IgG-specific cysteine protease to the antibody and/or antibody fragments contained in the sample is between 1:25 and 1:2500, preferably between 1:25 and 1:100. Another embodiment is characterized in that the

N-glycosidase F is derived from Flavobacterium meningosepticum (EC 3.2.2.18, EC 3.5.1.52). In another embodiment is the glycosidase Endo H and is employed at a pH between 6.0 and 6.5. In another embodiment the glycosidase has the amino acid sequence SEQ ID NO:2. Another embodiment is that the reducing agent is added simultaneously with formic acid and the incubation is in the presence of both agents. A further embodiment is that the incubation with the reduction agent is at a temperature of 60°C or more. In a further embodiment the mass spectrometry is an electrospray ionization time of flight mass spectrometry (ESI-TOF). In yet a further embodiment the liquid chromatography is a hydrophobic interaction chromatography or a $\pi$-$\pi$ interaction chromatography. In the case of a hydrophobic interaction chromatography the chromatography ligand in another embodiment is either a C8 or C18 ligand which is bound to a chromatography material having a pore size of 300 angstroms, or in the case of $\pi$-$\pi$ interaction chromatography the chromatography ligand is a diphenyl ligand. In a further embodiment either a Jupiter C18 column or a Zorbax 300SB C8 column or a Pursuit diphenyl column is used for the liquid chromatography. A Pursuit diphenyl column is used in another embodiment. In a further embodiment is the liquid chromatography in step c) a reverse phase chromatography. Another aspect of the invention is a method for detecting modified forms of an antibody wherein step c) is analysing the sample incubated under b) by means of a hydrophobic interaction chromatography.

The invention additionally encompasses the use of the IgG-specific cysteine protease IdeS for detecting IgG antibodies or IgG antibody fragments in a sample, characterized in that the sample is incubated with the IgG-specific cysteine protease IdeS and, after incubation with N-glycosidase F from Flavobacterium meningosepticum and the reducing agent trichloroethyl phosphate and formic acid, the fragments obtained are analysed by means of a coupled liquid chromatography and mass spectrometry.

An aspect of the present invention is also a kit for detecting antibodies or antibody fragments, characterized in that the kit contains

i) the IgG-specific cysteine protease IdeS from S. pyogenes,

ii) the endoglycosidase N-glycosidase F from Flavobacterium meningosepticum, and

iii) trichloroethyl phosphate and formic acid.

**Detailed description**

The present study is concerned with the analysis of degradation products and modifications of therapeutic, monoclonal antibodies, which are for example formed during the production of the antibody, the storage of the antibody, or by stress conditions during the formulation of the antibody.

A "polypeptide" is a polymer consisting of amino acids which are linked together by peptide bonds. It can either be produced enzymatically or synthetically. Polypeptides containing less than 20 amino acids are also referred to as "peptides".

A "protein" is a macromolecule which contains two or more polypeptides or it is a polypeptide which is composed of more than 100 amino acids. A protein can also contain non-peptidic components such as e.g. carbohydrates. Carbohydrates and other non-peptidic modifications are added by the cell which expresses the protein and therefore depend on the cell type. In this application proteins are defined by their amino acid sequence. Modifications such as carbohydrates are not explicitly described but may always be present.

The terms "antibody" and "immunoglobulin" that are used synonymously within this application describe a molecule which contains at least two light polypeptide chains (LC) and two heavy polypeptide chains (HC). Each of the light and heavy polypeptides contains a variable region (normally the amino terminus of the polypeptide) which contains binding domains for binding an antigen. Each of the heavy and light polypeptides contains a constant region (normally the carboxy terminus of the polypeptide) which for example is responsible for the binding of the antibody to cells. A light polypeptide or a light chain (LC) is normally composed of a variable domain $V_L$ and a constant domain $C_L$. A heavy polypeptide or a heavy chain (HC) is normally composed of a variable domain $V_H$ and a constant region which in turn is composed of the domains $C_H1$, hinge, $C_H2$, $C_H3$ and optionally $C_H4$. Antibodies may occur in numerous forms e.g. as Fv, Fab, and F(ab)$_2$ as well as single chains (scFv) (e.g. Huston, J.S., et al., Proc. Natl. Acad. Sci. USA 85 (1988) 5879-5883; Bird, R.E., et al., Science 242 (1988) 423-426; and Hood, L. E., et al., Immunology, Benjamin N.Y., 2nd Edition (1984) and Hunkapiller, T., and Hood, L., Nature 323 (1986) 15-16). Antibodies (immunoglobulins, Ig) are divided into various classes depending on the amino acid sequence of the constant region of the heavy chain of the antibody: IgA, IgD, IgE, IgG and IgM. Some of these classes are further subdivided into subclasses (isotypes) e.g. IgG into IgG1, IgG2, IgG3 and IgG4 or IgA into IgA1 and IgA2. The constant regions of the heavy chains are referred to as $\alpha$ (IgA), $\delta$ (IgD), $\varepsilon$ (IgE), $\gamma$ (IgG) and $\mu$ (IgM) depending on the class to which the antibody belongs.

General chromatographic methods are known to a person skilled in the art e.g. Chromatography, 5th edition, Part A: Fundamentals and Techniques, Heftmann, E. (ed.); Elsevier Science Publishing Company, New York, (1992); Advanced Chromatographic and Electromigration Methods in Biosciences, Deyl, Z. (ed.), Elsevier Science BV, Amsterdam, The Netherlands, (1998); Chromatography Today, Poole, D.F., and Poole, S.K., Elsevier Science Publishing Company, New York, (1991); Scopes, Protein Purification: Principles and Practice (1982); Sambrook, J., et al. (ed.), Molecular Cloning: A Laboratory Manual, Second Edition, Cold Spring Harbor Laboratory Press, Cold Spring Harbor, N.Y., 1989; or Current Protocols in Molecular Biology, Ausubel, F.M., et al. (eds.), John Wiley & Sons, Inc., New York.

Antibodies are biological macromolecules which may be subject to modification and degradation processes. These may be based on either enzymatic (catalytic) or non-enzymatic (non-catalytic) processes (Perkins, M., et al., Pharm. Res. 17 (2000) 1110-1117). Examples of non-enzymatic degradation reactions which often occur are described in the following.

## Oxidation of amino acids

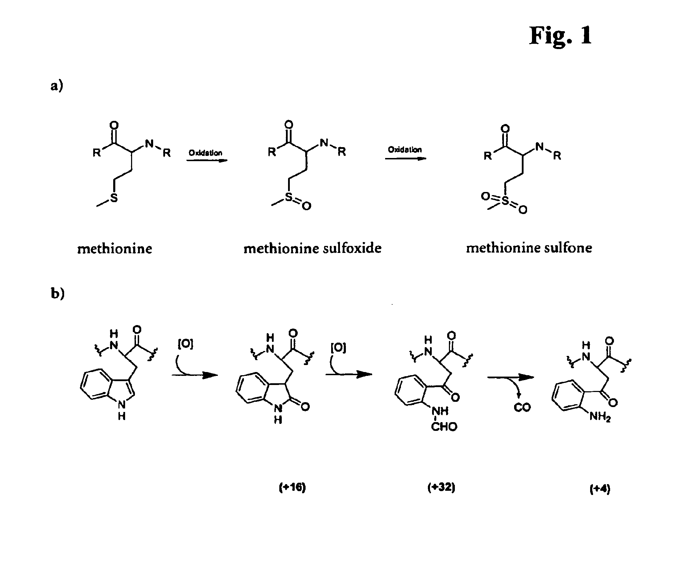

The oxidation of antibodies corresponds to a covalent modification of the amino acids of the heavy and light chain which is induced by reactive oxygen species. Even if almost all amino acids can, in principle, be oxidized, methionines (M) and tryptophans (W) are the most sensitive to oxidation. The oxidation of these amino acids (see figure 1) is of particular interest because this occurs in very many different proteins and often reduces or eliminates their biological activity, induces aggregation and promotes proteolysis (Houde, D., et al., J. Chromatogr. A 1123 (2006) 189-198). A simple oxidation of methionine to methionine sulfoxide results in a mass difference $\Delta m = +16$ Da for the oxidized species. Tryptophans are usually oxidized twice which results in a mass difference of $\Delta m = +32$ Da. In a further reaction the double oxidized form of tryptophan often rearranges to kynurenin resulting in a mass difference of $\Delta m = +4$ Da.

## Deamidation of amino acids

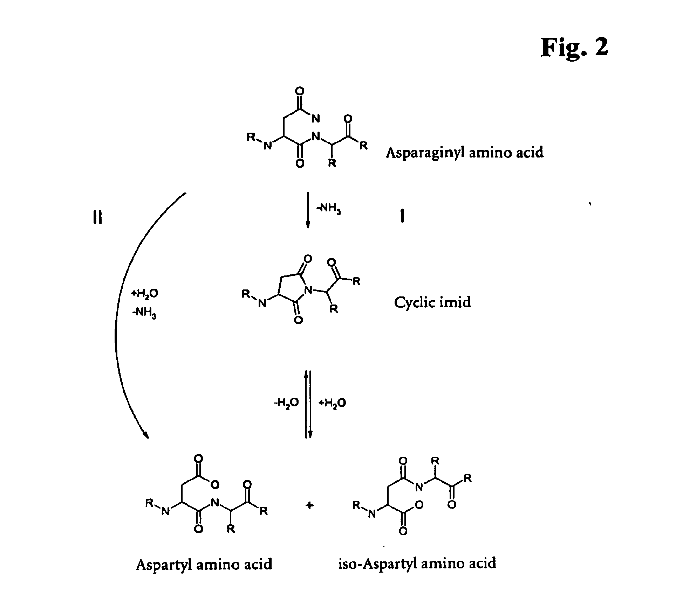

Deamidation of amino acids in antibody molecules can take place on asparagines (N) and glutamines (Q). However, it is usually asparagine which is affected. In addition certain amino acids sequences and amino acid combinations such as asparagine and glycine (NG), asparagine and serine (NS) and asparagines and threonine (NT) are particularly susceptible. The deamidation of asparagine is the main cause for the degradation of biological molecules during storage. Deamidations of the folded, intact antibody initially occur only slowly under increased stress conditions. Deamidations are facilitated when the three-dimensional structure of the antibody is destroyed such as for example after reductions and enzymatic cleavages because in these cases the amino acids are more accessible to reactions with the surrounding medium (figure 2). Asparagines can form cyclic amides (succinimides) in the form of an intermediate product which spontaneously hydrolyse to a mixture of isoaspartyl and aspartyl peptides in an approximate ratio of 3:1 (Chelius, D., et al., Anal. Chem. 77 (2005) 6004-6011). This reaction occurs preferentially at basic pH values. A mass difference $\Delta m = + 1$ Da for the deamidated species occurs as a result of a deamidation of asparagine to aspartate and isoaspartate. In the case of peptides and proteins which have a molecular weight of more than 10 kDa, it is impossible or very difficult to directly detect a mass difference of $\Delta m = + 1$ Da using the current mass spectrometers. Additionally a change of the charge distribution, to be more precise of the charge heterogeneity occurs.

**Formation of thioether bonds**

The formation of non-reducible thioether bridges is a phenomenon which is frequently observed with monoclonal antibodies, especially those of the subclass IgG1. This is based on the loss of a sulfur atom in the disulfide bridges which link the heavy and light chains of an antibody together or which intramolecularly stabilize the light and heavy chains. This modification of an antibody is favoured under increased stress conditions and in this case especially at elevated pH values. It is assumed that such a reaction is a β-elimination (Cohen, S.L., et al., J. Am. Chem. Soc., 129 (2007) 6976-6977). Since a sulfur atom is lost during the formation of a thioether bridge, this results in a mass difference of $\Delta m$ = -32 Da for the non-reduced, modified antibody species. In contrast to this is this disulfide bridge (S-S) cleaved into two SH groups during the reduction of an antibody. For this reason the resulting mass difference for reduced antibody components is $\Delta m$ = -34 Da.

**Formation of fragments**

Stress-induced fragmentation reactions are usually based on hydrolytic cleavages of the peptide bonds in the polypeptide chains of proteins e.g. of the heavy and light chain of an antibody. The proteolysis and thus the hydrolysis of peptide bonds can in principle take place between all amino acids, especially if steric tension or side chains of other amino acids favoring hydrolysis are present.

**Specific cleavages of antibodies**

Monoclonal antibodies are very large proteins and, furthermore, are extremely heterogeneous (microheterogenicity) due to the glycostructures of their heavy chains. In order to examine antibodies for the formation of degradation products and modifications it is expedient to cleave them into smaller fragments before the analysis. Therefore the cleavage of antibodies as part of the sample preparation is an important method for carrying out analytical investigations. In most cases the antibody is simply decomposed into its heavy and light chains by reduction of the disulfide bridges. In addition there are, however, also other methods for cleaving an antibody.

**Cleavage of disulfide bridges**

All disulfide bridges occurring in an IgG molecule can be cleaved by reduction. The free heavy and light chains of an antibody are obtained during the reduction. Tris-(2-carboxyethyl)-phosphine (TCEP) is a reducing agent that is often used because all disulfide bridges of an antibody are completely cleaved within a short period and the reduction occurs over the entire pH range (see e.g. Hau, J.C. and Hau, C.Y., Anal. Biochem. 220 (1994) 5-10). In one embodiment is the pH range of from 1.5 to 8.5. Dithiothreitol (DTT) is also characterized by a rapid cleavage of disulfide bridges. However, the DTT reduction in an acidic environment only proceeds very poorly. A denaturing step is usually necessary to complete the dissociation of the heavy and light chain. The denaturation additionally makes the disulfide groups more accessible. The denaturation can for example be carried out with the aid of guanidine/HCl or formic acid.

**Enzymatic cleavage**

Papain, a cysteine protease, cleaves peptide bonds relatively unspecifically after arginine (R), lysine (K), glutamic acid (E), histidine (H), glycine (G) and tyrosine (Y). If the incubation period is sufficiently long, the papain digestion leads to a total hydrolysis. However, antibodies can be cleaved relatively selectively in their hinge region by a limited proteolysis (Lottspeich, F., and Engels, J.W., "Bioanalytik Spektrum Akademischer Verlag" Munich 2nd Edition (2006) 201-214). The cleavage occurs on the N-terminal side of the disulfide bridges which connect the two heavy chains together. The disulfide bridges are retained in this process so that three fragments (2 Fab fragments, 1 Fc fragment) are obtained after the digestion. The two N-terminal fragments are referred to as antigen-binding fragments (Fab, antigen-binding fragment), the C-terminal fragment is referred to as the crystalline fragment (Fc, crystallizing fragment). Each Fab fragment is composed of a complete light chain and the amino-terminal half of the heavy chain. The Fc fragment is composed of the two carboxy-terminal halves of the heavy chains which are still linked together by the disulfide bridge.

**IdeS digestion**

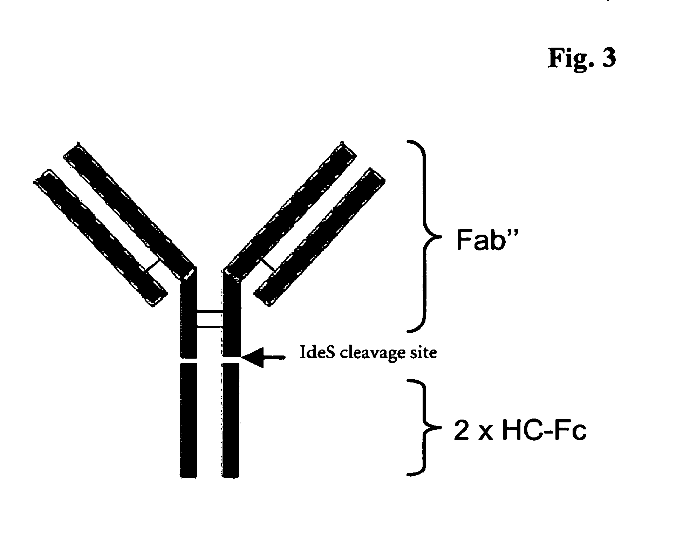

IdeS (immunoglobulin G-degrading enzyme of S. Pyogenes) is a cellular cysteine protease which can be isolated from the pathogenic bacterium Streptococcus pyogenes. This enzyme cleaves human IgG with high specificity directly before the recognition sequence GPSVFLFP. This sequence is located in the hinge region of IgG on the C-terminal side of disulfide bridges which link the two heavy chains (HC) together. The cleavage results in the C-terminal ends of the two heavy chains (2 HC-Fc fragments) and a Fab" fragment which results from the arms of the Fab fragments of the light and heavy chain that are linked by disulfide bridges (figure 3) (von Pavel-Rammingen, U., et al., EMBO Journal 21 (2002) 1607-1615).

If the IdeS fragments of the antibody are reduced with DTT or TCEP after the digestion, the two light chains of the antibody (2 LC) and the N-terminal fragments of the heavy chains (2 HC Fab) are obtained instead of the Fab" fragment. The C-terminal ends of the heavy chain (HC-Fc) are not affected by the reduction.

## Deglycosylation

The enzyme N-glycosidase F is a so-called endoglycosidase and completely cleaves off the sugar structures of glycoproteins, e.g. of the heavy chain of antibodies, between the polypeptide chain and the proximal N-acetyl glucosamine residue.

In order to simplify the very complex mass spectra that are sometimes obtained in the analysis of mixtures of various protein or peptide components by preseparating the components, mass spectrometry is often operated in combination with liquid chromatographic methods (LC/MS). For this purpose high performance HPLC systems are used among others to separate mixtures of dissolved substances according to their components before the mass spectrometric analysis. As a result of the chromatographic separation of an analyte mixture, the components leave the separating column at different times and in this manner can be analysed by mass spectrometry in the order of their elution.

A mass spectrum of each peak of the elution profile is obtained by coupling chromatography with mass spectrometry. The advantage of this is that one does not obtain a complex total spectrum of all components of the analyte solution but rather in the ideal case the homogeneous spectrum of a separated component.

The electrospray method (ESI) is a frequently used ionization method for converting dissolved molecules into gaseous ions in mass spectrometry. This is achieved by dispersing a liquid in an electrostatic field (electrospray). Very many small charged droplets which contain the analyte molecules are formed in this process.

The formation of highly charged ions is characteristic of the ESI process. Hence in the mass spectrum of a defined protein species one observes an entire series of ion signals each with a charge difference of $\Delta z = 1$ (as a rule by addition of one proton in the positive mode or subtraction of one proton in the negative mode) according to its molecular weight. The spectra of proteins show a characteristic approximately bell-shaped charge distribution of the molecular ions. The maximum of the distribution depends on parameters of the ESI mass spectrometer, on the pH of the solvent and on the state of denaturation of the protein. After cleavage of disulfide bridges and as a result of denaturation, proteins adopt a spatially more expanded structure so that more charges can be accepted (or released) by the molecule. The maximum of the charge distribution can therefore be shifted to higher (lower) charges.

The number of charges n of a multiply charged molecular ion and thus the molecular weight (M) can be calculated from the measured m/z ratios (m) of any two consecutive molecular ions ($m_2 > m_1$) of a charge distribution:

$$\text{formula 1:} \quad m_1 = \frac{M + nX}{n}$$

$$\text{formula 2:} \quad m_2 = \frac{M + (n-1)X}{(n-1)}$$

X in this connection is the mass of the charge carrier i.e. X = 1 (in the positive mode) for the addition of a proton and X = -1 (in the negative mode) for the subtraction of a proton. The value of n can be calculated by solving the variable n and equating formulae 1 and 2:

$$\text{formula 3:} \quad n = \frac{m_2 - X}{m_2 - m_1}$$

The molecular weight of the molecular ion can be calculated by solving formula 2 for M and by using the calculated result of n in formula 3:

$$\text{formula 4:} \quad M = n(m_1 - X)$$

The spectra are usually analysed with the aid of computer programs which can be used to determine the molecular weight either from all signals or from individual selected signals. So-called reconstructions are obtained as a result which show a given spectrum recalculated for a corresponding molecular weight range (deconvoluted spectra). The molecular weight can now be read off directly from the calculated peaks.

It was now surprisingly found that problems occurring with the previously used methods can be prevented by using a method according to the present invention. It was also surprisingly found that using the enzyme IdeS is advantageous for carrying out LC/MS analyses of antibodies.

Hence, the first aspect of the present invention is a method for detecting IgG antibodies and IgG antibody fragments in a sample characterized in that it comprises the following steps:

a) providing a sample which contains an IgG antibody and/or its cleavage products,

b) incubating the sample provided under a) with

i) the IgG-specific cysteine protease IdeS,

ii) N-glycosidase F

iii) the reducing agent trichloroethyl phosphate and formic acid,

whereby the incubation in steps b)-i), b)-ii) and b)-iii) is sequentially,

c) analysing the sample incubated under b) by means of a coupled liquid chromatography and mass spectrometry to detect the intact antibody and to detect fragments of the antibody contained in the solution provided under a).

The provided sample can, for example, be a solution containing the antibody, such as a reconstituted solution of a lyophilized antibody formulation. Modified antibody molecules have been formed in this solution during storage and lyophilization. These modifications are among others oxidation and deamidation of individual amino acids, formation of thioether bonds and the formation of antibody fragments.

The method according to the invention comprises the incubation of the sample with different agents. These agents are used to convert the antibody molecules and antibody fragment molecules contained in the sample into defined fragments. The first incubation step is the cleavage of the molecule with the IgG-specific cysteine protease IdeS, preferably the IdeS from Streptococcus pyogenes or Treponema denticola. In a further preferred embodiment the IgG-specific cysteine protease has the amino acid sequence SEQ ID NO: 1. The incubation with the IgG-specific cysteine protease takes place in one embodiment in a pH range between pH 5.5 and 8.5. In one embodiment the incubation is in the pH range of from pH 7.0 to 8.0. It was also found that the molar ratio of the IgG-specific cysteine protease to the antibody molecules (including the antibody fragment molecules) should be between 1:25 and 1:2500, in a preferred embodiment between 1:25 and 1:100.

The second incubation step is the cleavage of the carbohydrates from the antibody fragments with the aid of a glycosidase. The glycosidase is N-glycosidase F, also referred to as PNGase F. In one embodiment the N-glycosidase F is derived from Flavobacterium meningosepticum. In an embodiment the glycosidase is EC 3.2.218. In a further embodiment the glycosidase has the amino acid sequence SEQ ID NO: 2.

It is advantageous to firstly incubate the provided sample with the IgG-specific cysteine protease and subsequently to treat it with the glycosidase.

In the third step the disulfide bridges are cleaved by adding a reducing agent and preferably by adding trichloroethyl phosphate (TCEP). In one embodiment formic acid is added simultaneously with the addition of the reducing agent.

A combination of liquid chromatography and mass spectrometry is used to analyse the defined antibody fragments that are obtained. The individual fragments are separated by the liquid chromatography and can be subsequently determined by mass spectrometry. In one embodiment the mass spectrometry is an electrospray ionization-time-of-flight mass spectrometry (ESI-TOF). In one embodiment the liquid chromatography is a hydrophobic interaction chromatography or a π-π-interaction chromatography. In the case of a hydrophobic interaction chromatography, the chromatography ligand is in one embodiment either a C8 or C18 ligand which is located on a chromatography material with a pore size of 300 angstroms or in the case of π-π-interaction chromatography a diphenyl ligand is employed as the chromatography ligand. In one embodiment either a Jupiter C18 column or a Zorbax 300SB C8 column or a Pursuit diphenyl column, in a preferred embodiment a Pursuit diphenyl column is used for the liquid chromatography.

The invention additionally concerns the use of an IgG-specific cysteine protease IdeS from Streptococcus pyogenes for detecting IgG antibodies or IgG antibody fragments in a sample, characterized in that the sample is incubated with the IgG-specific cysteine protease and the fragments obtained are analysed after incubation with N-glycosidase F and the reducing agent trichloroethyl phosphate and formic acid by means of coupled liquid chromatography and mass spectrometry.

Another aspect of the present invention is a kit for detecting IgG antibodies or IgG antibody fragments, characterized in that the kit contains

i) the IgG-specific cysteine protease IdeS from S. pyogenes
ii) the endoglycosidase N-glycosidase F from Flavobacterium meningosepticum,
iii) trichloroethyl phosphate and formic acid.

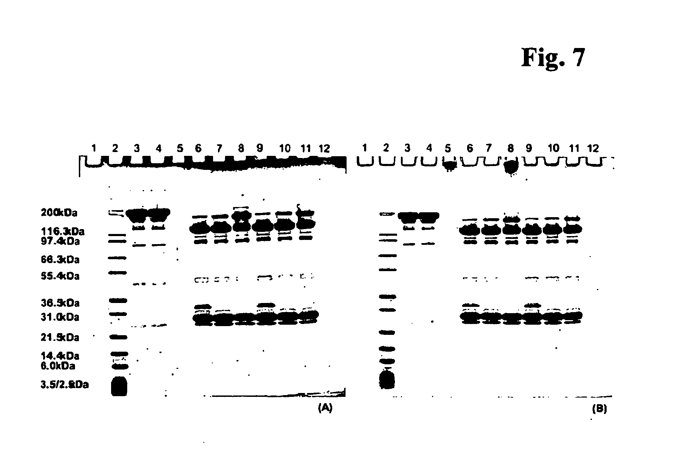

Firstly a reduction of the enzyme:antibody ratio and of the incubation time was tested in order to optimize the incubation conditions of the IdeS digestion. The shortest possible incubation time and smallest possible enzyme:antibody ratio were desirable for practical reasons because the presence of the enzyme component in the digested antibody analyte solution can lead to interfering signals in the mass spectrum that is obtained. The amount of enzyme used for the antibody digestion should be as small as possible in order to avoid this. At the same time it must be ensured that the antibody molecule is completely cleaved after the digestion. In order to additionally achieve the highest possible sample throughput and to optimize the experimental time course, the incubation period should be shortened while ensuring a complete digestion. The results of the gel electrophoretic separation of the digestion preparations (cf. figure 7) showed that two main bands (at about 100 kDa and about 25 kDa) which correspond to the expected products of IdeS cleavage were formed under all digestion conditions. The larger main band with a molecular weight of about 100 kDa corresponds to the expected "Fab part of the antibody which contains the two light chains (LC) and the Fab fragments of the heavy chain (HC). The smaller main band with a molecular weight of about 25 kDa is the expected Fc fragment of the HC. The results of the gel electrophoretic separation of the digestion preparations show that the enzyme:antibody ratio has a more significant effect on the IdeS digestion. With regard to the efficiency of the IdeS digestion of the various preparations, the results of the SDS-PAGE show that the band of the intact antibody (2HC/2LC) still occurs with a minimal intensity at an enzyme:antibody ratio of 1:50 and incubation periods of 0.5 h and 1 h (cf: figure 7A, lane 6 and 9). At the same enzyme:antibody ratio and incubations periods of 2 h and 5 h (cf: figure 7B, lane 6 and 9) the band of the intact antibody molecule has disappeared. Hence the preferred incubation period is between two and five hours and particularly preferably two hours. It has also turned out that an incubation period of 18 h and enzyme:antibody ratios of 1:50, 1:500, 1:2500, 1:10000 resulted in no difference compared to the five hour incubation at the same enzyme:antibody ratios. Thus an enzyme:antibody ratio of 1:25 to 1:100 is preferred. An enzyme:antibody ratio of 1:50 is particularly preferred.

The LC/MS-based method comprises a sample preparation method which is particularly suitable for separating and detecting stress-induced degradation products. In principle there are four possible variants of the sample preparation:

1. only the reduction of the antibody;

2. deglycosylation with subsequent reduction of the antibody;

3. IdeS digestion with subsequent reduction of the antibody;

4. deglycosylation with subsequent IdeS digestion and reduction of the antibody.

It has turned out that the preparation that has only been reduced (variant 1) and the IdeS-digested and reduced preparation (variant 3) are not suitable for the LC/MS analysis of the antibody solutions because heterogeneous spectra are obtained due to the glycostructures of the heavy chain which reduce the sensitivity of the LC/MS measurement and make the analysis more difficult.

It has now surprisingly turned out that the IdeS-digested, deglycosylated, reduced sample preparation is preferable to the deglycosylated, reduced sample preparation because smaller antibody fragments are obtained which have a positive effect on the mass resolution of the LC/MS measurement and allow modifications that may be additionally present to be located and allocated either to the HC-Fc or the HC-Fab part of the antibody. Furthermore, due to the cleavage of the heavy chain, it is possible to amplify the effect of stress-induced modifications that may have occurred on the chromatographic behaviour of the fragment in order to improve their separation from one another. No problems resulted from the simultaneous use of the two enzymes IdeS and N-glycosidase F.

It has turned out that for practical reasons it is particularly advantageous to firstly digest the antibody with IdeS and then to deglycosylate. The procedure was changed in this manner for practical reasons and had no significant effect on the results of the analyses. Formic acid is added at the same time as the addition of the TCEP solution, i.e. the formic acid and the TCEP solution are both added prior to the incubation, so both components are present during a single incubation.

In order to unequivocally and simply detect and quantify the formation of degradation products of antibody solutions, it is advantageous to separate the respective degradation species. The quality of the separation of an analyte solution is significantly influenced inter alia by the chromatographic properties of the stationary phase. The quality of the chromatographic separations was assessed on the basis of the peak resolution and peak sharpness of the respective column.

When selecting the columns further important parameters in addition to the polarity of the column matrix have to be taken into consideration for the separation such as e.g. the particle sizes should be as small as possible in order to achieve the highest possible plate numbers. In addition it should be borne in mind that antibodies are very large molecules. Consequently the pore size of the matrix particles also plays a decisive role when selecting a suitable column. The larger the pores of a matrix particle, the easier it is for antibody components to diffuse into the pores which improves the separation.

In comparison to the Jupiter C18 column, which was used as a reference column, no effective improvement of the separation of the degradation products was obtained with the Vydac C4 column under the employed chromatographic conditions. The Zorbax 300SB C8 has a different chromatographic selectivity than the Jupiter C18 column but also yields a comparable good result for the separation of the various antibody fragments. In contrast the Pursuit diphenyl column showed an improved separation result. The separation profile of this column exhibited more peaks and shoulders which occurred in the elution profile with a good resolution and acceptable peak sharpness, compared to the separation profile of the Jupiter C18 column. Therefore this column is preferred for the separation of degradation products of the antibody which were obtained according to the method of the invention.

It is intended to further elucidate the invention by the following examples, literature references and figures. The actual protective scope derives from the claims attached to this invention.

The invention is described in the following examples on the basis of an antibody example (mAb IGF-1R). This does not constitute a limitation of the invention but is rather only intended to illustrate it.

An example of a (preferably monoclonal) antibody is an antibody against the IGF-1 receptor (mAb IGF-1R) as described for example in WO 02/053596, WO 2004/071529, WO 2005/016967 WO 2006/008639, US 2005/0249730, US 2005/0084906, WO 2005/058967, WO 2006/013472, WO 2006/00181 US 2003/0165502, WO 2005/082415, WO 2005/016970, WO 03/106621, WO 04/083248, WO 2003/100008, WO 2004/087756, WO 2005/005635, WO 2005/094376 and WO 2007/115814.

**<u>Description of the figures:</u>**

| | |
|---|---|
| **Figure 1:** | Oxidation reaction of methionine (a) and tryptophane (b) as an example (see e.g. Taylor, S., et al., J. Biol. Chem. 278 (2003) 19587-19590). |
| **Figure 2:** | Deamidation of asparagine and isomerization to aspartate; route I is preferred under basic conditions (pH > 8), route II is preferred under acid conditions (pH < 5). |
| **Figure 3:** | Schematic representation of the IdeS digestion of IgG1 antibodies. |
| **Figure 4:** | Result of the gel electrophoretic separation of the antibody incubated at 40°C and stored at -80°C. |

        lane 1- sample buffer;
        lane 2 - molecular weight standard;
        lane 3 - sample buffer;
        lane 4 - standard, non reduced;
        lane 5 - antibody (-80°C), non reduced;
        lane 6 - antibody (30 days/ 40°C), non reduced;
        lane 7 - sample buffer;
        lane 8 - standard, reduced;
        lane 9 - antibody (-80°C) reduced;
        lane 10 - antibody (30 days/40°C), reduced;
        lane 11 - sample buffer;
        lane 12 - sample buffer.

| | |
|---|---|
| **Figure 5:** | Overlay of the SEC chromatograms of the stressed (1: 40 °C, 30 d) and of the non-stressed antibody (2: -80 °C) and of the placebo buffer (3) incubated at 40°C. |
| **Figure 6:** | Chromatograms of the ion exchange chromatography for the stressed (1: 40°C, 30 d) and non-stressed antibody (2: -80 °C) solution and the placebo buffer solution (3) incubated at 40°C. |

**Figure 7:** Separation of the antibody digested with IdeS at various ratios of enzyme to antibody (1:50 - 1:1250) and various incubation periods (0.5 h - 5h):
lane number - gelA - gelB:
1 - sample buffer - sample buffer;
2 - molecular weight standard - molecular weight standard;
3 - standard - standard;
4 - undigested antibody - undigested antibody;
5 - sample buffer - sample buffer;
6- 1:50, 0.5 h-1:50, 2.0 h;
7- 1:125,0.5 h-1:125, 2.0 h;
8 - 1:1250, 0.5 h - 1:1250, 2.0 h;
9-1:50,1.0 h-1:125, 5.0 h;
10 - 1:125, 1.0 h - 1:125, 5.0 h;
11 - 1:1250,1.0 h - 1:1250, 5.0 h;
12 - sample buffer - sample buffer.

**Figure 8:** Overlays of the elution profiles of the stressed and non-stressed antibodies on the Jupiter C18 standard column (A) and the Pursuit diphenyl column (B).

**Figure 9:** Overlays of the elution profiles of the stressed (1) and non-stressed (2) antibody on the Pursuit Diphenyl Column.

### Example 1

**Material and Methods**

The antibody which was used for the experiments in this investigation is a human, recombinant antibody of the IgG1 type. This antibody was expressed in CHO cells and purified by affinity chromatography and various ion exchange chromatography steps.

**Heat stress**

About 22 mg of the IgG1 antibody was rebuffered by dialysis in a 10 mM Tris/HCl buffer (pH 8.5). A portion of the rebuffered antibody solution was incubated at 40°C for 30 days. The other portion was frozen at -80°C as a control.

**Dialysis**

For the dialysis 1.5 ml antibody solution (c = 14.6 mg/ml) was transferred to a slide A-Lyzer dialysis cassette (capacity: 0.5 - 3 ml, molecular weight exclusion size: 10 kDa) and hung in the dialysis buffer. The buffer was continuously stirred during the dialysis and kept at about 8°C. The dialysis buffer solution was renewed several times during the dialysis in order to ensure the completest possible exchange of the buffer solutions. Table 1 shows the time scheme for the exchange of the buffer solution.

**Table 1:Dialysis scheme for rebuffering the antibody solution**

| volume$_{dialysis\ buffer}$ [L] | time [h] | temperature [°C] |
| --- | --- | --- |
| 1.5 | 3 | 8 |
| 1.5 | 15 | 8 |
| 1.5 | 3 | 8 |

After completion of the dialysis, the antibody solution was transferred from the slide A-Lyzer cassette into a 5 ml reaction vessel. The volume of the dialysed antibody solution was determined by weighing on an analytical balance (AT261 Delta Range, Mettler-Toledo).

**Sterile filtration**

After the dialysis the antibody solution was sterilized by filtration through a Minisart single syringe filter (0.2 μm, Sartorius) under low germ level conditions. The sampling for the determination of the antibody concentration was also carried out under low germ level conditions.

## Determination of the antibody concentration

The antibody concentration was determined by means of an absorption measurement at 280 nm on a spectral photometer of the type Uvikon XL (Goebel Company). The extinction coefficient of the antibody that was used was 1.55 mL·mg$^{-1}$·cm$^{-1}$ and was calculated according to the method of Pace, C.N., et al., (Protein Sci. 4 (1995) 2411-2423).

## SDS PAGE

The antibody solutions were separated by gel electrophoresis using a Power Ease 300 electrophoresis station as well as gels, buffers and other reagents from the Invitrogen Company. A Tris-glycine 4-20 % gradient gel was used for the separation. The Tris-glycine SDS running buffer (10x) which was diluted 1:10 (v/v) before use with twice distilled water, was used as the running buffer.

The antibody solutions that had been incubated at 40°C and those that had been stored at -80°C were applied under reducing as well as under non-reducing conditions. 5 μl of a Mark 12™ protein marker was also applied to the gel as a reference in order to determine the relative molecular weight of the samples to be examined. The electrophoretic separation was carried out at a voltage of 125 V and a run time of about 95 min. After the gel electrophoresis the gels were stained in Simply Blue Safe Stain (Coomassie G 250 staining solution) according to the manufacturer's instructions. Subsequently the gel was destained in twice distilled water.

The stained gels were preserved using Dry-Ease mini cellophanes and a drying solution (10 % glycerol (v/v), 40 % methanol (v/v), 10 % acetic acid (v/v), 40 % water (v/v)). For this the gels were incubated for 5 min in the drying solution and incubated for a further 10 min after adding two cellophanes. Afterwards the gels were placed clear of bubbles between the cellophanes and dried in a clamping frame.

For the non-reducing SDS-PAGE the antibody solutions were diluted to a concentration of about 1 mg·ml$^{-1}$ with 10 mM Tris/HCl buffer (pH 8.5). Subsequently 10 μl (corresponding to 10 μg antibody) of the diluted antibody solutions was admixed with 10 μl SDS sample buffer (2x) in a ratio of 1:2 (v/v), mixed and finally briefly centrifuged. The samples were denatured for 10 min at 70°C. Afterwards they were again briefly centrifuged. For the electrophoretic separation 16 μl (corresponding to 8 μg antibody) of the denatured samples were applied to the gel. In addition to the protein marker, an antibody reference standard (c = 16.5 mg·ml$^{-1}$) diluted to a concentration of 1 mg·ml$^{-1}$ and subsequently treated in the same way as the antibody samples was applied as a reference.

For the reducing SDS-PAGE the antibody solutions were also diluted with 10 mM Tris/HCl buffer (pH 8.5) to a concentration of 1 mg·ml$^{-1}$. DTT (dithiothreitol) was used to reduce the solutions. In order to ensure a complete reduction of the antibody, the DTT solution was prepared at a concentration of 100 mM in SDS sample buffer (2x). Subsequently 10 μl (corresponding to 10 μg antibody) of the diluted solutions was admixed in a ratio of 1:2 (v/v) with 10 μl 2x SDS sample buffer + 100 mM DTT, mixed and briefly centrifuged. The samples were denatured for 10 min at 70°C. Afterwards they were again briefly centrifuged. For the electrophoretic separation 16 μl (corresponding to 8 μg antibody) of the denatured samples were applied to the gel. In addition to the protein marker an antibody reference standard (c = 16.5 mg·ml$^{-1}$) diluted to a concentration of 1 mg·ml$^{-1}$ and subsequently treated in the same way as the antibody samples was applied as a reference.

## Size exclusion chromatography (SEC)

The TSK gel G3000 SWXL gel filtration column (7.8 x 300 mm; 5 μm, 250 A) from the Tosoh Bioscience Company was used to chromatographically separate the components of the antibody solutions according to size. The separation was carried out on a Shimadzu-HPLC system. The sample components were eluted isocratically with a 200 mM potassium phosphate, 250 mM potassium chloride buffer (pH 7.0) over a period of 30 min and at a flow rate of 0.5 ml·min$^{-1}$. The temperature of the TSK gel G3000 SWXL gel filtration column was kept constant at 25°C during the separation. The temperature of the autosampler was 6°C. The sample components were detected at a wavelength of 280 nm using a diode array detector.

## Ion exchange chromatography (IEC)

The weak cation exchange column SynChropak WCX (4.6 x 250 mm, 6 μm, 300A) from the Agilent Company was used for the ion chromatographic separation of the components of the antibody solutions. The separation was carried out on a Shimadzu HPLC system. The sample components were eluted with the aid of a binary gradient system of eluant A (10 mM sodium phosphate buffer, pH 7.0) and eluant B (10 mM sodium phosphate buffer, 750 mM sodium chloride buffer, pH 7.0) at a flow rate of 1 ml·min$^{-1}$ and a run time of 55 minutes. The elution profile is shown in table 2.

The temperature of the SynChropak WCX cation exchanger column was kept constant at 25°C during the separation. The temperature of the autosampler was 6°C. The sample components were detected at a wavelength of 280 nm using a diode array detector.

**Table 2: Profie of the gradient for separating the antibody solution by means of IEC**

| time (min) | eluant A (%) | eluant B (%) |
|---|---|---|
| 0.0 | 95 | 5 |
| 12.0 | 80 | 20 |
| 17.0 | 80 | 20 |
| 35.0 | 70 | 30 |
| 40.0 | 70 | 30 |
| 41.0 | 0 | 100 |
| 44.0 | 0 | 100 |
| 45.0 | 95 | 5 |
| 55.0 | 95 | 5 |

### Example 2

**Heat stress**

In order to accelerate the formation of degradation products and of modifications of the monoclonal antibody, it was subjected to a defined heat stress in an incubator (Venticell, MM-Medcenter). For the incubation the dialysed antibody solution was incubated for 30 days at 40°C. In addition 250 $\mu$l of the antibody solution was divided into 10 $\mu$l aliquots and stored at -80°C. These served as the zero point control.

After 30 days the aliquots subjected to the heat stress were removed from the incubator and examined visually for infestation with microorganisms. The removed aliquots were pooled in a reaction vessel in order to obtain a homogeneous, stressed analytical solution. The homogenization was carried out by carefully mixing the antibody solution (MS2 minishaker, IKA, Staufen). The homogenized antibody solution was divided into 20 $\mu$l aliquots and stored at -80°C.

For the analysis by liquid chromatography and mass spectrometry, about 22 mg antibody (c = 14.6 mg·ml$^{-1}$) was rebuffered in a 10 mM Tris/HCl buffer (pH 8.5) and subsequently incubated for 30 days at 40°C.

**Preparation of a heat-incubated placebo buffer solution**

In order to ensure in later analyses that observed changes of the antibody solution that result from storage at 40°C are not due to changes in the buffer solution, two 15 ml aliquots of the 10 mM Tris/HCl buffer were sterilized by filtration under low germ level conditions and also incubated in the incubator (Venticell, MM-Medcenter) for 30 days at 40°C. The subsequent procedure was carried out as for the antibody solution.

**Analysis of the heat-incubated solution by gel electrophoresis**

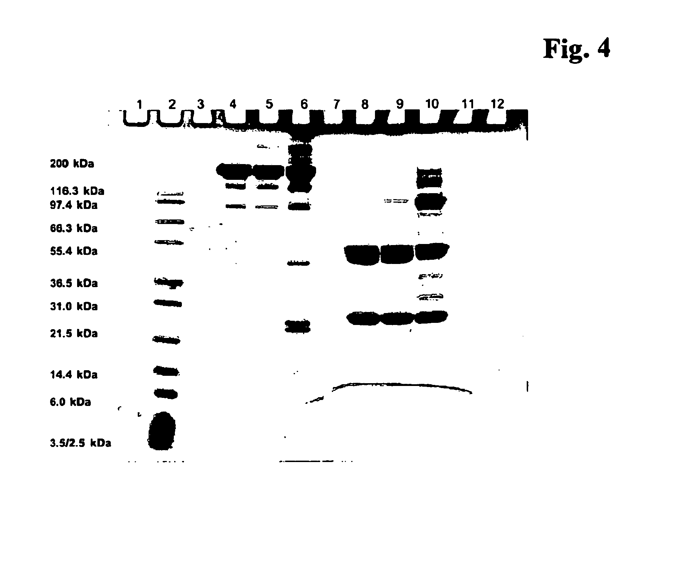

The result of the gel electrophoretic separation of the antibody incubated at 40°C and stored at -80°C is shown in figure 4. The incubation at 40°C results in the formation of aggregates as well as of fragments of the antibody molecule (lane 6 and 10). The non-incubated antibody samples show the typical band pattern of an IgG1 molecule (cf. lane 4 and 5) under non-reducing conditions. The main band corresponds to the intact antibody consisting of two heavy chains (HC) and two light chains (LC). In addition bands can be seen for antibody species whose structure has not been completely preserved. These bands are the 2HC/LC species which lacks a light chain, the half antibody (HC/LC), the free heavy chain (HC) and the free light chain (LC) (cf. lane 4 and 5).

This typical band pattern is also basically seen in the non-reduced antibody sample incubated at 40°C (cf. lane 6). The main band is again the intact antibody (2 HC/2LC). In addition bands occur for the various non-intact antibody species (2HC/LC, HC/LC, HC, LC) at a higher intensity than the antibody sample stored at -80°C. In addition further bands are formed in the stressed sample in the range from about 90 kDa to > 200 kDa (cf. lane 6). The species which occur below the main band are antibody fragments and those above the main band are aggregates.

Two bands which appear to be of particular interest for analysis by means of LC/MS occur in the region of about 21 kDa (cf. lane 6). This is a non-covalent Fab fragment in which the HC Fab fragment (AA 1-220) and the LC are not kept together by a disulfide bridge but rather by non-covalent interactions (ionic interactions, hydrogen bonds, dipole-dipole interactions, van-der-Waals forces). Since the samples are applied to the gel under denaturing conditions, HC Fab and LC appear as single bands in the gel (cf. lane 6). In this connection the upper band in the gel is LC (LC*) and the lower band is the HC Fab fragment (Cohen, S.L., et al., J. Am. Chem. Soc., 129 (2007) 6976-6977). The remaining antibody which is composed of the second Fab fragment and of the Fc fragment can be allocated to the band which occurs at a weak intensity in the region of 97 kDa and 116 kDa (cf. lane 6).

The main bands in the separation under reducing conditions are the heavy (HC) and the light chain (LC) (cf. lane 8, 9 and 10). In addition to the two main bands, two further bands occur in the non-stressed antibody samples in the range between about 97 kDa and 120 kDa with a lower intensity. These are covalent, non-reducible aggregates (cf. lane 8 and 9).

The antibody solution incubated at 40°C shows the same band pattern as the non-incubated antibody solution (cf. lane 10). The two bands which can be allocated to the non-reducible species in the case of the non-stressed sample do not occur with a very much greater intensity in the stressed sample (cf. lane 10). In addition to the already mentioned species (HC, LC and the two covalent, non-reducible bands) a large number of additional bands occur in the range of about 30 kDa to 200 kDa in the stressed antibody solution. These are reduced covalent aggregates, non-reducible species and fragments of the reduced species (cf. lane 10).

The band of the non-reducible aggregate species which occurs in the gel at about 90 kDa to 95 kDa and which can be allocated to the half antibody is of particular interest for the LC/MS analysis. The intensity of this band increases significantly as a result of the incubation. The increase in intensity at this band is most likely due to the formation of a thioether species. This species is a half antibody molecule with an actual mass of 75 kDa whose HC and LC are not linked together by a disulfide bridge but rather by a non-reducible thioether bridge (Tous, G.I., et al., Anal. Chem. 77 (2005) 2675-2682).

## Analysis of the heat-incubated solution by means of SEC

Size exclusion chromatography (SEC) gives an overview of the formation of stress-induced fragments and aggregates. Since SEC is carried out under native conditions, covalent as well as non-covalent aggregates are detected in the analysis. In order to establish a connection between the formation of degradation products and the incubation at 40°C, the non-stressed sample which was stored at -80°C is also analysed in addition to the stressed antibody solution.

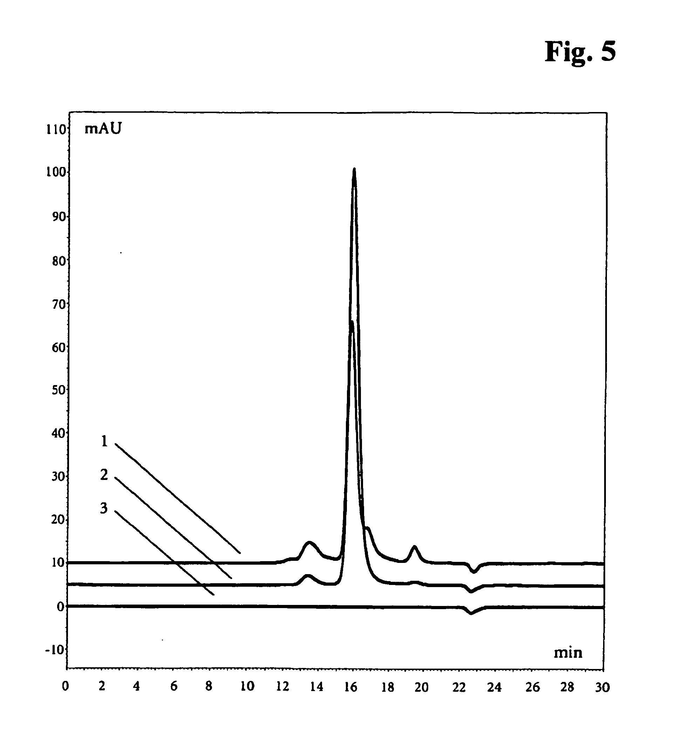

Figure 5 shows the result of the SEC for the placebo buffer solution incubated at 40°C, the antibody incubated at 40°C and the antibody stored at -80°C. 50 $\mu$l or 50 $\mu$g antibody were applied in each case for the analysis. The results of the analysis of the molecular weight standard served as a reference for determining the molecular weight sizes of the components of the antibody solutions. They are listed in table 4. Table 3 shows the percentages of the various antibody species in the elution profile. The calculated percentages are based on the proportion of the respective area of the peak of a certain species relative to the sum of the area of all peaks which occur.

### Table 3: Evaluation of the SEC for the stressed and the non-stressed antibody

| | high molecular weight species (RT ~ 12 - 14.5 min) [%] | main peak (RT ~ 15 - 16.5 min) [%] | shoulder in the low-molecular weight range (RT ~ 16.5 - 18 min) [%] | low-molecular weight species (RT 19-20 min) [%] |
|---|---|---|---|---|
| -80°C | 3.19 | 95.81 | - | 1.00 |
| 40°C/30 days | 12.33 | 72.20 | 10.85 | 4.62 |

### Table 4: Results of the chromatographic separation of the SEC molecular weight standard

| Substance | mass [kDa] | retention time [min] |
|---|---|---|
| thyroglobulin | 670 | 11.9 |
| gamma globulin | 158 | 15.8 |
| ovalbumin | 44 | 18.5 |
| myoglobin | 17 | 20.4 |
| vitamin B12 | 1.35 | 23.4 |

The elution profile of the stressed antibody species correlates with the results of the SDS-PAGE. The results of the SEC also show that aggregates have been formed and that the antibody has been degraded during the incubation (cf. figure 5).

The main species with a retention time of about 15 - 16.5 min. which can be assigned to the intact antibody, decreases during the incubation at 40°C from about 96 % to 72 % of the total area (cf. table 3). The retention time of the main species (RT 15 - 16.5 min) corresponds to a molecular weight of about 150 kDa based on the molecular weight standard (cf. table 4). The aggregates with a molecular weight of > 158 kDa (cf. table 4) elute in a time window of about 12 - 14.5 minutes. The peak with this elution time increases as a result of the incubation at 40°C from about 3 % to about 12 % of the total area (cf. table 3). A clear shoulder forms in the decreasing region of the peak of the main species (RT about 16.5 - 18 min.) in the elution profile of the stressed antibody which has an area proportion of about 11 % and which does not occur to this extent in the antibody solution stored at -80°C (cf. figure 5). The antibody species which elute in the region of the shoulder have a molecular weight of about 70 kDa to 120 kDa (cf. table 4). These are species such as the half antibody (HC/LC), antibodies with a missing light chain (2HC/LC) and Fab + Fc fragments. A further peak is additionally seen at a retention time of about 19 - 20 min. which can be allocated a molecular weight between about 20 kDa and 40 kDa (cf. table 4). The heavy (HC) and the light chain (LC) as well as the HC Fab of the non-covalent Fab fragment elute in this time window. This peak increases as a result of the 40°C incubation from about 1 % to 5 % of the total area (cf. table 3).

The comparison of the elution profile of the placebo buffer incubated at 40°C with the antibody solution incubated at 40°C shows that no peaks occur as a result of the incubation of the buffer.

### Analysis of the heat-incubated solution by means of IEC

Ion exchange chromatography (IEC) is suitable for detecting changes in charge which are due to modifications of the antibody molecule.

In order to be able to identify stress-related changes in the antibody, the non-stressed antibody stored at -80°C is applied in addition to the antibody incubated at 40°C.

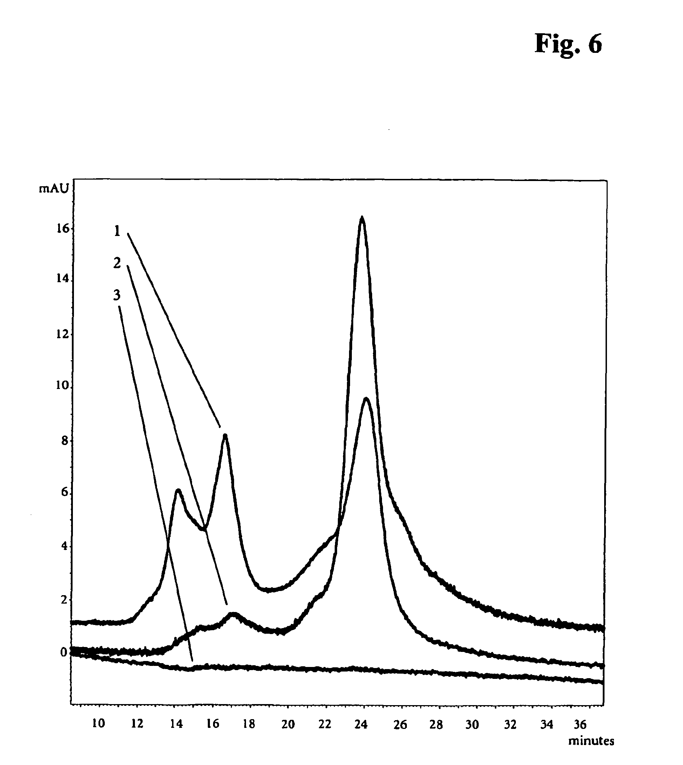

Figure 6 shows the chromatograms obtained in the ion exchange chromatography for the stressed and for the non-stressed antibody solution and for the placebo buffer solution incubated at 40°C. 50 $\mu$l or 25 $\mu$g antibody was applied in each case for the analysis. The percentages of the various species of the elution profile are shown in table 5. The calculated percentages refer to the proportions of the respective area of the peak of a certain species relative to the sum of the area of all peaks that occur.

**Table 5: Evaluation of the IEC for the stressed and the non-stressed antibody**

| | acidic range (PRT ~ 12 - 18 min) [%] | shoulder in the acidic range (RT ~ 19 - 22 min) [%] | main peak (RT ~22 - 25 min) [%] | shoulder in the basic range (RT ~ 26 - 32 min) [%] |
|---|---|---|---|---|
| - 80°C | 7.69 | 4.52 | 60.75 | 27.04 |
| 40°C/30 days | 43.83 | 16.57 | 39.60 | 0 |

A comparison of the elution profiles of the antibodies stored at -80°C and the antibodies incubated at 40°C shows that a significant shift in charge into the acidic range was induced by the incubation (cf. figure 6). Thus the intensity of the peak of the main species (retention time RT about 22 - 25 min) that can be allocated to the intact antibody decreased significantly from about 61 % to about 40 % of the total area (cf. table 5), whereas the intensity of the peaks in the acidic range of the elution profile (RT 12 - 18 min) increased significantly (increase from about 8 % to about 44 % of the total area). The shoulder which is seen in the basic range of the non-stressed antibody sample (RT 26 - 32 min) completely disappears during the incubation at 40°C (cf. figure 6). In return the shoulder in the acidic part of the main peak (RT 19 - 22 min) increases in intensity in the case of the stressed antibody solution. In this case the area increases from about 5 % to about 17 % of the total area (cf. table 5). A comparison of the elution profile of the placebo buffer incubated at 40°C with the antibody solution incubated at 40°C shows that no peaks occur as a result of the incubation of the buffer.

Charge shifts into the acidic range which are induced by the incubation of an antibody are usually mainly due to deamidation reactions. In deamidations an amino group is replaced by a hydroxyl group which introduces an additional negative charge into the molecule. The result of the IEC chromatogram shows that deamidations and other changes in charge have occurred to a high degree as a result of the incubation at 40°C.

## Example 3

### Optimization of the IdeS digestion

The enzyme IdeS was present in a 50 mM Tris/HCl buffer (pH 8.0) at a concentration of 1 mg/ml. A dilution series comprising three different IdeS concentrations was prepared for the enzyme addition to the digestion solution. The final concentration of IdeS in the predilutions (V) was : 0.1 mg·ml$^{-1}$ (V 1), 0.01 mg·ml$^{-1}$ (V 2) and 0.001 mg·ml$^{-1}$ (V 3). The dilution was carried out using 50 mM Tris/HCl buffer (pH 8.0).

The antibody to be analysed was present at a concentration of 15.5 mg/ml in a histidine-buffered solution, pH 6.0. The antibody solution was diluted to a concentration of c = 5 mg·ml$^{-1}$ with 50 mM Tris/HCl buffer (pH 8.0) for the addition to the digestion solution.

The pipetting scheme for the preparation for the digestion solutions is listed in table 6.

**Table 6: Pipetting scheme for the preparation of the IdeS digestion solutions.**

| Ratio enzyme: | IdeS [μg] | IdeS dilution [μl] | antibody [μg (μl)] | 50 mM Tris buffer [μl] | antibody in the digestion |
|---|---|---|---|---|---|
| no enzyme | | | 50 (10) | 40 | 1 μg/μl |
| 1:50 | 1.00 | 10 (V 1) | 50 (10) | 30 | 1 μg/μl |
| 1:125 | 0.40 | 40 (V 2) | 50 (10) | 0 | 1 μg/μl |
| 1:1250 | 0.04 | 40 (V 3) | 50 (10) | 0 | 1 μg/μl |

The prepared digestion solutions were incubated for 0.5, 1.0, 2.0 and 5.0 hours at 37°C. An antibody solution to which no enzyme was added was prepared as a zero control. For the control solution 10 μl (50 μg) antibody was added by pipette to 40 μl 50 mM Tris/HCl buffer (pH 8.0). The incubation was carried out for 5.0 h at 37°C.

In addition an antibody reference standard solution (antibody in 20 mM histidine, 240 mM trehalose, 0.02 % Tween 20, pH = 6.0, c = 16.5 mg·ml$^{-1}$) was diluted to a concentration of 1 mg·ml$^{-1}$ with 50 mM Tris/HCl buffer (pH 8.0) as an additional control. The reference standard solution was applied without prior incubation.

The various antibody digestion solutions were applied under non-reducing conditions.

## Example 4

### Comparison of different sample preparation methods

### Only reduced:

43 μg Antibody was reduced with 0.43 M trichloroethyl phosphate (0.5 M in H$_2$O) at a concentration of c = 1 mg·ml$^{-1}$. For this purpose 38 μl TCEP solution (0.5 M in H$_2$O) was added to 5 μl of the non-stressed antibody solution (c = 8.55 mg·ml$^{-1}$) and incubated for 30 min at 37°C. Subsequently the antibody solution was diluted with formic acid (1 %, v/v) at a ratio 1:2 (v/v). After dilution with formic acid the concentration of the antibody was 0.5 mg·ml$^{-1}$. The proportion of formic acid was 0.5 % (v/v). The solution obtained was centrifuged (Minispin, Eppendorf) for 2 min at 13,400 rpm. The supernatant was carefully removed with a pipette and transferred to an analytical tube. 4 μl (2 μg) antibody was applied to the column in the LC/MS experiments.

### IdeS digestion and reduction:

Digestion with IdeS was carried out by incubating 43 μg antibody at a concentration of 1 mg·ml$^{-1}$. For this purpose 5 μl of the non-stressed antibody solution (c = 8.55 mg·ml$^{-1}$) was diluted with 34 μl 50 mM Tris/HCl buffer (pH 8.0), 4 μl IdeS solution (0.25 mg·ml$^{-1}$ in 50 mM Tris/HCl buffer (pH 8.0)) was added and it was incubated for 2 h at 37°C. Afterwards it was reduced with 0.25 M TCEP (0.5 M in H$_2$O) at a concentration of 0.5 mg·ml$^{-1}$. For this purpose 35 μl of the digested antibody solution (c = 1 mg·ml$^{-1}$) was added to 35 μl TCEP solution (0.5 M in H$_2$O) and incubated for 30 min at 37°C. Subsequently the antibody solution was diluted with formic acid (1 %, v/v) at a ratio 1:2 (v/v). After dilution with formic acid the concentration of the antibody was 0.25 mg·ml$^{-1}$. The proportion of formic acid was 0.5 %. The antibody solution obtained was centrifuged (Minispin, Eppendorf) for 2 min at 13,400 rpm. The supernatant was carefully removed with a pipette and transferred to an analytical tube. 8 μl (2 μg) antibody was applied to the column in the LC/MS experiments.

## Example 5

**LC-MS analytical method**

The antibody was firstly digested with IdeS, then deglycosylated and subsequently reduced for the LC/MS analyses for characterizing the degradation products of the antibody.

**IdeS digestion**

For the digestion with IdeS, 4 $\mu$l IdeS solution (c = 0.25 mg·ml$^{-1}$ in 50 mM Tris/HCl buffer, pH 8.0) was added in each case to 7.9 $\mu$l of the stressed antibody (c = 6.26 mg·ml$^{-1}$) and to 5.8 $\mu$l of the non-stressed antibody (c = 8.55 mg·ml$^{-1}$). The solution was diluted with 50 mM Tris/HCl buffer (pH 8.0) to an antibody concentration of 1 mg·ml$^{-1}$ and incubated for 2 h at 37°C. The enzyme: antibody ratio was 1:50.

**Deglycosylation with N-glycosidase F**

The glycostructures of the heavy chain of the antibody were cleaved off with the aid of the enzyme N-glycosidase F. The N-glycosidically bound sugars were cleaved after the IdeS digestion by incubation at an antibody concentration of 0.5 mg·ml$^{-1}$ in 50 mM Tris/HCl buffer (pH 8.0). The cleavage was initiated by adding 0.5 $\mu$l (activity: 10 U·ml$^{-1}$) N-glycosidase F. The incubation was carried out for 4 h at 37°C.

**Reduction - Example 1**

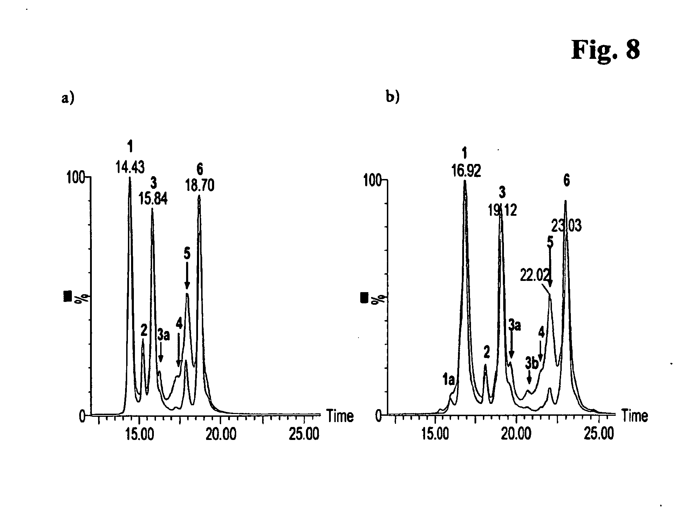

The reduction was carried out after the IdeS digestion and deglycosylation by adding 60 $\mu$l TCEP (trichloroethyl phosphate; 0.5 M in H$_2$O). Subsequently the mixture was diluted with formic acid (1 %, v/v) at a ratio of 1:2 (v/v) so that a concentration of 0.16 mg·ml$^{-1}$ was obtained for the antibody in the reduction solution. The concentration of TCEP was 0.1 M, the proportion of formic acid in the solution was 0.5 % (w/v). The incubation was carried out in the presence of both formic acid and TCEP for 30 min at 37°C. Subsequently the antibody solution that was obtained was centrifuged for 2 min at 13400 rpm (Minispin, Eppendorf). The supernatant was carefully removed by pipette and transferred to an analytical tube. 13 $\mu$l (2 $\mu$g) antibody was applied to the column in the LC/MS experiment (figure 8).

**Reduction - Example 2**

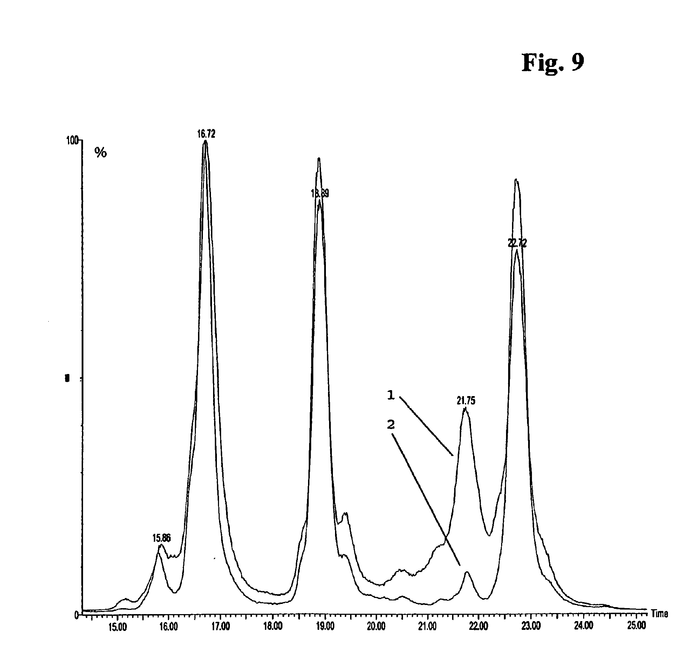

The reduction was carried out after the IdeS digestion and deglycosylation by adding 60 $\mu$l TCEP (trichloroethyl phosphate; 0.5 M in H$_2$O). Subsequently the mixture was diluted with formic acid (1 %, v/v) at a ratio of 1:2 (v/v) so that a concentration of 0.16 mg·ml$^{-1}$ was obtained for the antibody in the reduction solution. The concentration of TCEP was 0.1 M, the proportion of formic acid in the solution was 0.5 % (w/v). The incubation was carried out in the presence of both formic acid and TCEP for 10 min at 70°C. Subsequently the antibody solution that was obtained was centrifuged for 2 min at 13400 rpm (Minispin, Eppendorf). The supernatant was carefully removed by pipette and transferred to an analytical tube. 13 $\mu$l (2 $\mu$g) antibody was applied to the column in the LC/MS experiment (figure 9).

**RP-HPLC separation procedure**

The RP separation of the preparations was carried out on an Agilent 1100 Cap-LC system equipped with a micro degasser, a capillary pump, a micro autosampler with a temperature control unit, a column oven and a multi wavelength detector (Agilent, Waldbronn). A Jupiter C18 column (Phenomenex, Aschaffenburg) of 3.5 $\mu$m particle size, 300 A pore size and having the dimensions 1.0 x 250 mm was used for the standard separation which represents the reference point. The chromatographic separation was carried out at 75 °C and at a flow rate of 40 $\mu$l·min$^{-1}$. Two $\mu$g antibody were applied to the column for each preparation. The sample components were eluted with a binary gradient that was mixed together from eluant A (0.5 % formic acid in H$_2$O (v/v)) and eluant B (70 % 2-propanol, 20 % acetonitrile, 9.5 % H$_2$O and 0.5 % formic acid (v/v)). Table 7 shows the profile of the gradient used for the elution.

**Table 7: Gradient profile for the RP separation**

| Time [min] | Eluant B [%] | Slope [eluant B·min$^{-1}$] |
|:---:|:---:|:---:|
| 0 | 20 | - |
| 7 | 20 | 0 |

(continued)

| Time [min] | Eluant B [%] | Slope [eluant B·min⁻¹] |
|---|---|---|
| 9 | 25 | 2.50 |
| 29 | 50 | 1.25 |
| 32 | 100 | 16.67 |
| 37 | 100 | 0 |
| 38 | 20 | -80 |
| 50 | 20 | 0 |

The eluted sample components were detected with the aid of a UV-detector at a wavelength of 280 nm. For the online TOF mass analysis, the HPLC system was coupled to a micromass LCT mass spectrometer (Waters, Eschborn). A blank value was recorded by injecting 10 μl eluant A.

**Mass spectrometric analysis**

The online ESI-TOF mass analysis of the eluate of the LC separation was carried out on a micromass LCT mass spectrometer (Waters, Eschborn) which was equipped with an electrospray ion source. The data were recorded in a mass range of 600-2000 amu (atomic mass units) in the positive mode (ES⁺) at a cone temperature of 80°C and a desolvation temperature of 100°C. The capillary voltage was 3000 V, the cone voltage was 30 V, the RF lens voltage was 400 V and the extraction cone voltage was 1 V. The other parameter settings for the ESI-TOF mass analysis are summarized in table 8.

**Table 8: Parameter settings of the LCT mass spectrometer**

| Parameter | Settings |
|---|---|
| aperture (V) | 2.0 |
| acceleration (V) | 200 |
| focus (V) | 0.0 |
| steering (V) | 0.7 |
| ion energy (V) | 29.0 |
| tube lens (V) | 28.0 |
| TOF flight tube (V) | 4600.0 |
| reflectron (V) | 1770.0 |
| cone gas flow (L/hr) | 64 |
| desolvation gas flow (L/hr) | 392 |
| Resolution | 4000.0 |
| lock mass | 0.0000 |
| mass window +/- | 1.0000 |

The one point calibration (LTeff determination) was carried out using 0.085 % $H_3PO_4$ in 50 % acetonitrile which was fed into the mass spectrometer at a flow rate of 5 μl·min⁻¹ with the aid of a Hamilton pump (pump 11, Harvard Apparatus). The mass accuracy was ~ 100 ppm. The data were evaluated with the aid of the Mass Lynx 4.0 data recording software.

### Example 6

**Comparison of the mass spectra of the Pursuit diphenyl column with those of the Jupiter C18 column**

As with the Jupiter C18 column, the Pursuit diphenyl column only enabled a partial separation of the oxidized HC-Fc species (23778 Da) in a preshoulder of peak 1 (cf. figure 8). Also no improvement of the separation was achieved for the pyroglutamate species of the light chain of the antibody. It eluted in the Jupiter C18 column as well as in the Pursuit diphenyl column in peak 3a with a comparable resolution. The HC-Fab fragment (amino acids (AA) 1-220) also co-eluted in the two columns with the intact HC-Fab species in the last peak of the elution profile. Thus it was also not possible to separate this fragment in a discrete peak in the separation using the Pursuit diphenyl column. A comparison of the mass profiles of the Jupiter C18 and of the Pursuit diphenyl column additionally showed that the mass of peak 3b of the Pursuit diphenyl column correlated with peak 4 of the Jupiter column. Both showed a mass of 33630 Da which can be allocated to the IdeS enzyme. In addition peak 5 of the Pursuit DP column correlates with peak 5 of the Jupiter C18 column. In both cases the masses of the thioether-containing HC-Fab-LC complex (48918 Da), the mass of the incompletely reduced HC-Fab fragment (25377 Da) and the mass of N-glycosidase F (34783 Da) occur.

In contrast peak 4 of the Pursuit diphenyl column does not correlate with any peak of the Jupiter C18 column and thus constitutes a difference to the elution profile of the Jupiter C18 column. This peak exhibits the mass 48916 Da which can be allocated to the thioether species and the mass 49118 Da. This mass corresponds to the molecular weight of the heavy chain of the antibody. The mass 49118 Da thus represents the intact heavy chain of the antibody that is uncleaved by IdeS which has been separated from the remaining antibody molecule by reduction during the course of sample preparation.

Differences in the comparison of the elution profile of the two columns are also seen in the case of peak 1a which is not detectable in the elution profile of the Jupiter C18 column. A species having a mass of 20037 Da is separated in peak 1a of the Pursuit diphenyl column. It is possible to allocate this to a HC fragment which has been formed as a result of an Asp-Pro cleavage between aspartic acid 271 (D271) and proline 272 (P272). The theoretical mass of the C-terminal fragment of the Asp-Pro cleavage has a value of 20033 Da. Since this mass occurs in the mass spectrum of the stressed as well as in the mass spectrum of the unstressed antibody, this cleavage product is not a degradation product that is obtained as a result of the 40°C incubation. Although this fragment is not explicitly formed by the 40°C incubation, it is nevertheless a degradation product of the antibody which can only be separated by the Pursuit diphenyl column. In the case of the Jupiter C18 column this fragment occurs together with the oxidized species in the shoulder of peak 1 which indicates a better separation performance of the Pursuit diphenyl column.

A comparison between the Pursuit diphenyl and the Jupiter C18 column shows that the Pursuit diphenyl column has better separation properties than the Jupiter C18 column in special respects (cf. peak 1a and peak 4) and has comparable results in other regards. Thus it was possible to effectively improve the separation of the antibody species.

## Claims

1. Method for detecting IgG antibodies and IgG antibody fragments in a sample, **characterized in that** it comprises the following in vitro steps:

   a) providing a sample which contains an IgG antibody and/or IgG antibody fragments,
   b) incubating the sample provided under a) with

       i) the IgG-specific cysteine protease IdeS,
       ii) N-glycosidase F, and
       iii) the reducing agent trichloroethyl phosphate and formic acid, whereby the incubation in steps b)-i), b)-ii) and b)-iii) is sequentially,

   c) analysing the sample incubated under b) by means of a coupled liquid chromatography and mass spectrometry to detect the intact antibody and/or to detect fragments of the antibody contained in the sample provided under a).

2. Method according to claim 1, **characterized in that** the IgG-specific cysteine protease has the amino acid sequence SEQ ID NO: 1.

3. Method according to one of the claims 1 to 2, **characterized in that** the glycosidase has the amino acid sequence SEQ ID NO: 2.

4. Method according to one of the previous claims, **characterized in that** the liquid chromatography is a reverse phase liquid chromatography.

5. Method according to claim 4, characterized that the reverse phase chromatography employs a chromatography material with C8 or C18 residues.

6. Method according to one of the claims 1 to 3, **characterized in that** the liquid chromatography is a hydrophobic interaction chromatography.

7. Method according to claim 6, **characterized in that** the hydrophobic interaction chromatography employs a chromatography material with diphenyl residues.

8. Use of the IgG-specific cysteine protease IdeS from Streptococcus pyogenes for detecting IgG antibodies or IgG antibody fragments in a sample, **characterized in that** the sample is incubated with the IgG-specific cysteine protease and, after incubation with N-glycosidase F from Flavobacterium meningosepticum and the reducing agent trichloroethyl phosphate and formic acid, the fragments obtained are analysed by means of a coupled liquid chromatography and mass spectrometry.

9. Kit for detecting IgG antibodies or IgG antibody fragments, **characterized in that** the kit contains

       i) the IgG-specific cysteine protease IdeS from S. pyogenes,
       ii) the glycosidase N-glycosidase F from Flavobacterium meningosepticum, and
       iii) trichloroethyl phosphate and formic acid.

## Patentansprüche

1. Verfahren zum Nachweis von IgG-Antikörpern und IgG-Antikörperfragmenten in einer Probe, **dadurch gekennzeichnet, dass** die folgenden Schritte umfasst sind:

   a) Bereitstellen einer Probe, die einen IgG-Antikörper und/oder IgG-Antikörperfragmente enthält,
   b) Inkubieren der Probe, die unter a) bereitgestellt wird, mit

       i) der IgG-spezifischen Cystein-Protease IdeS,
       ii) N-Glycosidase F, und
       iii) dem Reduktionsmittel Trichlorethylphosphat und Ameisensäure, wobei die Inkubationsschritte b)-i), b)-ii) und b)-iii) aufeinander folgend sind,

c) Analysieren der Probe, die unter b) inkubiert wurde, mittels gekoppelter Flüssigkeitschromatographie und Massenspektrometrie zum Nachweis des intakten Antikörpers und/oder von Fragmenten des Antikörpers in der Probe, die unter a) bereitgestellt wurde.

2. Verfahren nach Anspruch 1, **dadurch gekennzeichnet, dass** die IgG-spezifische Cystein-Protease die Aminosäuresequenz SEQ ID NO: 1 hat.

3. Verfahren nach einem der Ansprüche 1 bis 2, **dadurch gekennzeichnet, dass** die Glycosidase die Aminosäuresequenz SEQ ID NO: 2 hat.

4. Verfahren nach einem der vorherigen Ansprüche, **dadurch gekennzeichnet, dass** die Flüssigkeitschromatographie eine Umkehrphasen-Flüssigkeitschromatographie ist.

5. Verfahren nach Anspruch 4, **dadurch gekennzeichnet, dass** in der Umkehrphasen-Flüssigkeitschromatographie ein Chromatographiematerial mit C8- oder C18-Resten verwendet wird.

6. Verfahren nach einem der Ansprüche 1 bis 3, **dadurch gekennzeichnet, dass** die Flüssigkeitschromatographie eine hydrophobe Wechselwirkungs-Chromatographie ist.

7. Verfahren nach Anspruch 6, **dadurch gekennzeichnet, dass** die hydrophobe Wechselwirkungs-Chromatographie ein Chromatographiematerial mit Diphenyl-Resten verwendet.

8. Verwendung der IgG-spezifischen Cystein-Protease IdeS von Streptococcus pyogenes zum Nachweis von IgG-Antikörpern oder IgG-Antikörperfragmenten in einer Probe, **dadurch gekennzeichnet, dass** die Probe mit der IgG-spezifischen Cystein-Protease inkubiert wird und, nach Inkubation mit N-Glycosidase F von Flavobacterium meningosepticum und dem Reduktionsmittel Trichlorethylphosphat und Ameisensäure, die erhaltenen Fragmente mittels gekoppelter Flüssigkeitschromatographie und Massenspektrometrie analysiert werden.

9. Kit zum Nachweis von IgG-Antikörpern oder IgG-Antikörperfragmenten, **dadurch gekennzeichnet, dass** der Kit beinhaltet:

i) die IgG-spezifische Cystein-Protease IdeS von S. pyogenes,
ii) die Glycosidase N-Glycosidase F von Flavobacterium meningosepticum, und
iii) Trichlorethylphosphat und Ameisensäure.

**Revendications**

1. Procédé de détection d'anticorps IgG et de fragments d'anticorps IgG dans un échantillon, **caractérisé en ce qu'**il comprend les étapes suivantes:

a) fourniture d'un échantillon qui contient un anticorps IgG et/ou des fragments d'anticorps IgG,
b) incubation d'un échantillon fourni en a) avec

i) la cystéine-protéase IdeS spécifique des IgG,
ii) une N-glycosidase F, et
iii) l'agent réducteur phosphate de trichloroéthyle et acide formique, l'incubation dans les étapes b)-i), b)-ii) et b)-iii) s'effectuant de façon séquentielle,

c) analyse de l'échantillon mis à incuber dans b) au moyen d'une chromatographie liquide couplée à une spectrométrie de masse pour détecter l'anticorps entier et/ou détecter des fragments de l'anticorps contenu dans l'échantillon fourni dans a).

2. Procédé selon la revendication 1, **caractérisé en ce que** la cystéine-protéase spécifique des IgG a la séquence d'aminoacides SEQ ID NO: 1.

3. Procédé selon l'une des revendications 1 à 2, **caractérisé en ce que** la glycosidase a la séquence d'aminoacides SEQ ID NO: 2.

4. Procédé selon l'une des revendications précédentes, **caractérisé en ce que** la chromatographie liquide est une chromatographie liquide en phase inverse.

5. Procédé selon la revendication 4, **caractérisé en ce que** la chromatographie en phase inverse utilise une substance chromatographique ayant des résidus en C8 ou C18.

6. Procédé selon l'une des revendications 1 à 3, **caractérisé en ce que** la chromatographie liquide est une chromatographie à interaction hydrophobe.

7. Procédé selon la revendication 6, **caractérisé en ce que** la chromatographie à interaction hydrophobe utilise une substance chromatographique ayant des résidus diphényle.

8. Utilisation de la cystéine-protéase IdeS spécifique des IgG de *Streptococcus pyogenes* pour la détection d'anticorps IgG ou de fragments d'anticorps IgG dans un échantillon, **caractérisée en ce que** l'on met l'échantillon à incuber avec la cystéine-protéase spécifique des IgG et, après incubation avec la N-glycosidase F de *Flavobacterium meningosepticum* et l'agent réducteur phosphate de trichloroéthyle et acide formique, on analyse les fragments obtenus au moyen d'une chromatographie liquide couplée à une spectrométrie de masse.

9. Kit de détection d'anticorps IgG ou de fragments d'anticorps IgG, **caractérisé en ce que** le kit contient

   i) la cystéine-protéase IdeS spécifique des IgG de *S. pyogenes,*
   ii) la glycosidase N-glycosidase F de *Flavobacterium meningosepticum,* et
   iii) du phosphate de trichloroéthyle et de l'acide formique.

# Fig. 1

a)

methionine           methionine sulfoxide          methionine sulfone

b)

(+16)           (+32)           (+4)

# Fig. 2

Asparaginyl amino acid

Cyclic imid

Aspartyl amino acid          iso-Aspartyl amino acid

**Fig. 3**

Fab"

IdeS cleavage site

2 x HC-Fc

**Fig. 4**

**Fig. 5**

**Fig. 6**

**Fig. 7**

(A)

(B)

**Fig. 8**

a)

b)

**Fig. 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070237784 A
- EP 1458861 A
- WO 2006131347 A
- WO 02053596 A
- WO 2004071529 A
- WO 2005016967 A
- WO 2006008639 A
- US 20050249730 A
- US 20050084906 A
- WO 2005058967 A
- WO 2006013472 A
- WO 200600181 A

- US 20030165502 A
- WO 2005082415 A
- WO 2005016970 A
- WO 03106621 A
- WO 04083248 A
- WO 2003100008 A
- WO 2004087756 A
- WO 2005005635 A
- WO 2005094376 A
- WO 2007115814 A
- EP 07024863 A

**Non-patent literature cited in the description**

- **YAN, B. et al.** *J. Chromatog. A,* 2007, vol. 1164, 153-161
- **DILLON, T.M. et al.** *J. Chromatogr. A,* 2004, vol. 1053, 299-305
- **VINCENTS, B. et al.** *Biochem.,* 2004, vol. 43, 15540-15549
- **VON PAWEL-RAMMINGEN, U. et al.** *EMBO J.,* 2002, vol. 21, 1607-1615
- **HESS, J.K. et al.** *J. Microbiol. Meth.,* 2007, vol. 70, 284-291
- **SHANTHA RAJU, T. ; SCALLON, B.J.** *Biochem. Biophys. Res. Commun.,* 2006, vol. 341, 797-803
- **BECK, A. et al.** *J. Chrom. B,* 2005, vol. 819, 2303-218
- **LOO, T. et al.** *Prot. Expr. Purif.,* 2002, vol. 24, 90-98
- **HUSTON, J.S. et al.** *Proc. Natl. Acad. Sci. USA,* 1988, vol. 85, 5879-5883
- **BIRD, R.E. et al.** *Science,* 1988, vol. 242, 423-426
- **HOOD, L. E. et al.** Immunology. 1984
- **HUNKAPILLER, T. ; HOOD, L.** Nature. 1986, vol. 323, 15-16
- Part A: Fundamentals and Techniques. Chromatography. Elsevier Science Publishing Company, 1992
- Advanced Chromatographic and Electromigration Methods in Biosciences. Elsevier Science, 1998

- **POOLE, D.F. ; POOLE, S.K.** Chromatography Today. Elsevier Science Publishing Company, 1991
- **SCOPES.** Protein Purification: Principles and Practice. 1982
- Molecular Cloning: A Laboratory Manual. Cold Spring Harbor Laboratory Press, 1989
- Current Protocols in Molecular Biology. John Wiley & Sons, Inc,
- **PERKINS, M. et al.** *Pharm. Res.,* 2000, vol. 17, 1110-1117
- **HOUDE, D. et al.** *J. Chromatogr. A,* 2006, vol. 1123, 189-198
- **CHELIUS, D. et al.** *Anal. Chem.,* 2005, vol. 77, 6004-6011
- **COHEN, S.L. et al.** *J. Am. Chem. Soc.,* 2007, vol. 129, 6976-6977
- **HAU, J.C. ; HAU, C.Y.** *Anal. Biochem.,* 1994, vol. 220, 5-10
- **LOTTSPEICH, F. ; ENGELS, J.W.** Bioanalytik Spektrum Akademischer Verlag. 2006, 201-214
- **VON PAVEL-RAMMINGEN, U. et al.** *EMBO Journal,* 2002, vol. 21, 1607-1615
- **TAYLOR, S. et al.** *J. Biol. Chem.,* 2003, vol. 278, 19587-19590
- **PACE, C.N. et al.** *Protein Sci.,* 1995, vol. 4, 2411-2423
- **TOUS, G.I. et al.** *Anal. Chem.,* 2005, vol. 77, 2675-2682